(19) Europäisches Patentamt / European Patent Office / Office européen des brevets

(11) **EP 3 394 776 B1**

(12) **EUROPEAN PATENT SPECIFICATION**

(45) Date of publication and mention
of the grant of the patent:
**12.11.2025 Bulletin 2025/46**

(21) Application number: **16823031.6**

(22) Date of filing: **21.12.2016**

(51) International Patent Classification (IPC):
**G16B 20/20** (2019.01)     **C12Q 1/6883** (2018.01)
**C12Q 1/6827** (2018.01)

(52) Cooperative Patent Classification (CPC):
**C12Q 1/6827; C12Q 1/6883; G16B 20/00**

(86) International application number:
**PCT/GB2016/054019**

(87) International publication number:
**WO 2017/109487 (29.06.2017 Gazette 2017/26)**

(54) **DETECTION OF CHROMOSOME ABNORMALITIES**

NACHWEIS VON CHROMOSOMENANOMALIEN

DÉTECTION D'ANOMALIES CHROMOSOMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2015 GB 201522665**

(43) Date of publication of application:
**31.10.2018 Bulletin 2018/44**

(73) Proprietor: **Yourgene Health UK Ltd
Manchester M15 6SH (GB)**

(72) Inventor: **FORMAN, Matthew Charles
Leicester
Leicestershire LE2 6DW (GB)**

(74) Representative: **HGF
HGF Limited
1 City Walk
Leeds LS11 9DX (GB)**

(56) References cited:
**US-A1- 2010 112 590     US-A1- 2015 267 255**

- **A. J. SEHNERT ET AL: "Optimal Detection of
Fetal Chromosomal Abnormalities by Massively
Parallel DNA Sequencing of Cell-Free Fetal DNA
from Maternal Blood", CLINICAL CHEMISTRY,
vol. 57, no. 7, 1 July 2011 (2011-07-01), pages
1042 - 1049, XP055035090, ISSN: 0009-9147, DOI:
10.1373/clinchem.2011.165910**
- **H. CHRISTINA FAN ET AL: "Sensitivity of
Noninvasive Prenatal Detection of Fetal
Aneuploidy from Maternal Plasma Using
Shotgun Sequencing Is Limited Only by
Counting Statistics", PLOS ONE, vol. 5, no. 5, 1
January 2010 (2010-01-01), pages e10439,
XP055026436, ISSN: 1932-6203, DOI: 10.1371/
journal.pone.0010439**

**Description**

Background

The invention relates to the detection of chromosomal abnormalities. In particular, the invention relates to determining a probability of fetal chromosomal abnormality from a biological sample.

Downs syndrome is a relatively common genetic disorder. The syndrome is caused by the presence of an extra chromosome 21 (trisomy 21 or T21) or less frequently an extra substantial portion of that chromosome. Other trisomies are also known such as T13 or T18. Methods of prenatal diagnosis of such conditions are known which are based on DNA sequencing of DNA molecules from maternal plasma. Reliably determining chromosome abnormality from the biological sample is problematic given variables associated with processing of the DNA.

It is an object of embodiments of the invention to at least mitigate one or more of the problems of the prior art.

"Optimal Detection of Fetal Chromosomal Abnormalities by Massively Parallel DNA Sequencing of Cell-Free Fetal DNA from Maternal Blood", 1st July 2011 (2011-07-01), by A. J. Sehnert et al. discloses a sequence tag mapping and chromosome-quantification method for the detection of multiple chromosomal abnormalities.

US 2010/112590A1 discloses methods, systems, and apparatus for determining whether a fetal chromosomal aneuploidy exists from a biological sample obtained from a pregnant female.

US 2015/267255A1 discloses a method of detecting chromosomal abnormalities, in particular, the invention relates to the diagnosis of fetal chromosomal abnormalities such as trisomy 21 (Down's syndrome) which comprises sequence analysis of cell-free DNA molecules in plasma samples obtained from maternal blood during gestation of the fetus.

"Sensitivity of Noninvasive Prenatal Detection of Fetal Aneuploidy from Maternal Plasma Using Shotgun Sequencing Is Limited Only by Counting Statistics", 1st January 2010 (2010-01-01), by H. Christina Fan et al. discloses a method to computationally remove GC bias in short read sequencing data by applying weight to each sequenced read based on local genomic GC content.

Brief Description of the Drawings

Embodiments of the invention will now be described by way of example only, with reference to the accompanying figures, in which:

Figure 1 shows an illustration of an apparatus according to an embodiment of the invention;

Figure 2 shows a flow chart illustrating a method according to an embodiment of the invention;

Figure 3 shows a representation of data forming a model according to an embodiment of the invention;

Figure 4 shows a representation of data forming a model according to an embodiment of the invention;

Figure 5 shows density curves for models at a specific chromosome sequence density according to an embodiment of the invention;

Figure 6 shows density curves for models at a different specific chromosome sequence density according to an embodiment of the invention; and

Figure 7 illustrates density contours for first and second models for a target chromosome.

Detailed Description of Embodiments of the Invention

Embodiments of the invention are set out in the appended claims. Further embodiments are defined in the corresponding dependent claims. Furthermore, the description and drawings present additional examples, aspects, and non-claimed embodiments for the better understanding of the claimed invention.

Embodiments of the invention relate to determining a probability of a fetal chromosomal abnormality. In embodiments of the invention a likelihood ratio is determined which is indicative of fetal chromosomal abnormality for a target chromosome. The likelihood ratio is determined based upon first and second models, as will be explained. In detecting such abnormalities it is important to ensure, as much as possible, that false results are not determined. In particular, it is particularly desired to reduce a probability of a false negative result being determined. However it is also important to ensure that data is efficiently used. That is that results are generated in an acceptable number of cases or tests. A test result should, ideally, be declared where possible, rather than a test indicating that the result is unreliable due to one or more parameters associated with the test. For example, use of a predetermined cut off value associated with a test parameter might cause a test result not to be generated or to be indicated as unreliable, thereby causing the test to be repeated or a further biological sample to be required.

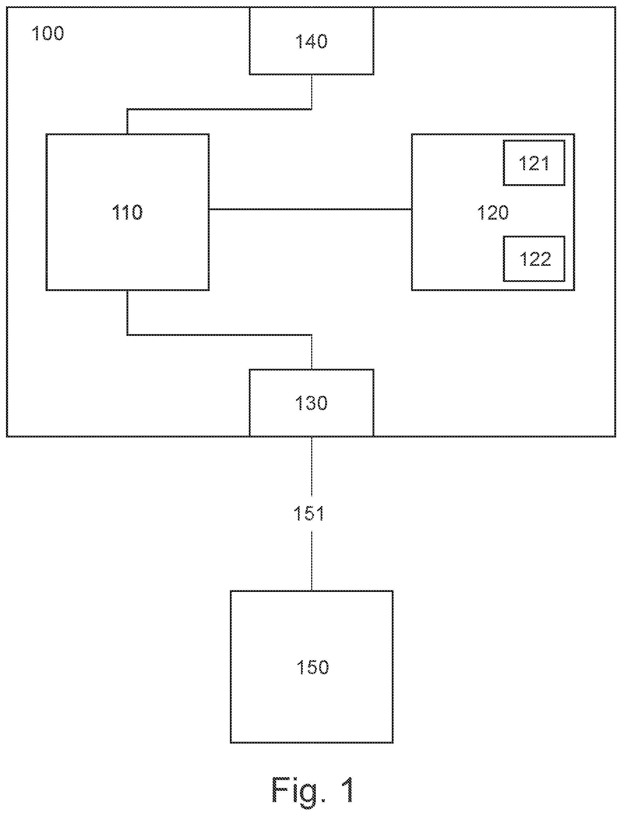

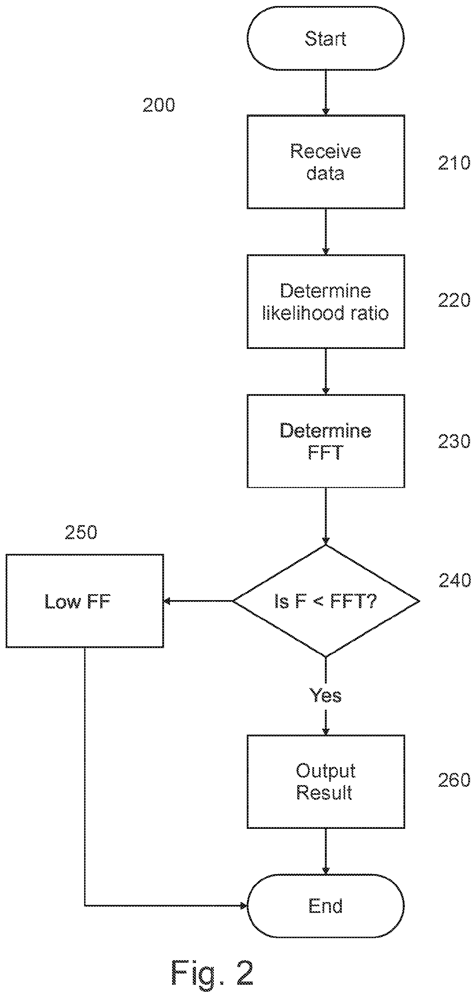

Figure 1 illustrates an apparatus 100 according to an embodiment of the invention. The apparatus 100 comprises a processing unit 110 which is communicably coupled to a memory unit 120. The processing unit 110 is operable to execute instructions which form computer-executable code or software. The processing unit 110 may comprise one or more processors. The processing unit 110 is operable according to the computer-executable code to perform operations on data determined from a biological sample, as will be explained. The computer-executable code may be arranged to perform a method according to an embodiment of the invention which will be explained with reference to Figure 2. The memory unit 120 may be formed by one or more memory devices such as one or more of ROM and RAM devices. The memory unit 120 is accessible by the processing unit 110 to read stored data and to store data in the memory unit 120. The computer executable code may be stored in the memory unit 120. The memory unit 120 may store data representing first and second models 121, 122 as will be explained.

The apparatus 100 further comprises an interface unit 130 for receiving data. In particular the data is received from a DNA processing system 150. A data communication path 151 may exist between the interface unit 130 and DNA processing system 150. The communication path 151 may comprise one or more communications networks including the internet. The apparatus 100 may further comprise an output unit 140 for outputting data indicative of a result, as will be explained.

Figure 2 illustrates a method 200 according to an embodiment of the invention. The method 200 is a method of determining a probability of a fetal chromosomal abnormality.

The method 200 comprises a step 210 of determining data indicative of a first parameter for a target chromosome and a second parameter representative of chromosome sequence density from a biological sample obtained from a female subject. The data may be determined by receiving the data from the DNA processing system 150. The data may be received by the interface unit 130 via the communication path 151.

By way of illustration reference will be made to WO2014/033455 which discloses a method of detecting chromosomal abnormalities. Whilst reference is made to WO '455, it will be realized that embodiments of the present invention are not limited in this respect.

In WO '455 sequence data is obtained for nucleic acid molecules within a biological sample and a matching analysis is performed between each nucleic acid sequence within the sequence data and a sequence which corresponds to a unique portion of a reference genome, such that each matched nucleic acid is assigned to a particular chromosome, or a part of said chromosome, within the reference genome. A ratio of the total number of matched nucleic acids assigned to a target chromosome relative to the total number of matched nucleic acids assigned to each of one or more reference chromosomes is measured. A statistically significant difference in the measured ratio, relative to the ratio in a normal pregnancy, is indicative of a fetal abnormality in the target chromosome. The target chromosome may correspond to T21, although it may also correspond to other chromosomes.

The first parameter for the target chromosome may, in some embodiments, be indicative of the total number of matched nucleic acids assigned to one or more target chromosomes, such as one or more of chromosome 21, chromosome 18 or chromosome 13, although it will be appreciated that other chromosomes may be used.

In some embodiments the first parameter may correspond to a chromosome count ratio i.e. the ratio of the total number of matched nucleic acids assigned to each target chromosome relative to the total number of matched nucleic acids assigned to each of one or more reference chromosomes. However, in some embodiments the ratio may be calculated by the apparatus 100 based on the second parameter, as will be explained.

The second parameter representative of chromosome sequence density from the biological sample may, in some embodiments, be indicative of the total number of matched nucleic acids assigned to each of one or more reference chromosomes. In some embodiments, the second parameter may be an autosome fragment count number. The autosome fragment count number is a number of autosome fragments counted by the DNA processing system 150. In other embodiments the second parameter may be a measure of sequencing coverage of the reference chromosome. In one embodiment the second parameter may be indicative of a mean depth of coverage.

The method 200 comprises a step 220 of determining a likelihood ratio indicative of fetal chromosomal abnormality. The likelihood ratio may be determined as a ratio between a probability of chromosomal abnormality and a probability of chromosomal normality according to respective models 121, 122. The respective models may be stored in the memory unit 120.

As noted above, the memory unit 120 of the apparatus 100 stores data representing first and second models 121, 122. The first model 121 is indicative of a probability of chromosome abnormality. The second model 122 is indicative of chromosome normality. The first and second models 121, 122 are each parameterized based upon the first and second parameters.

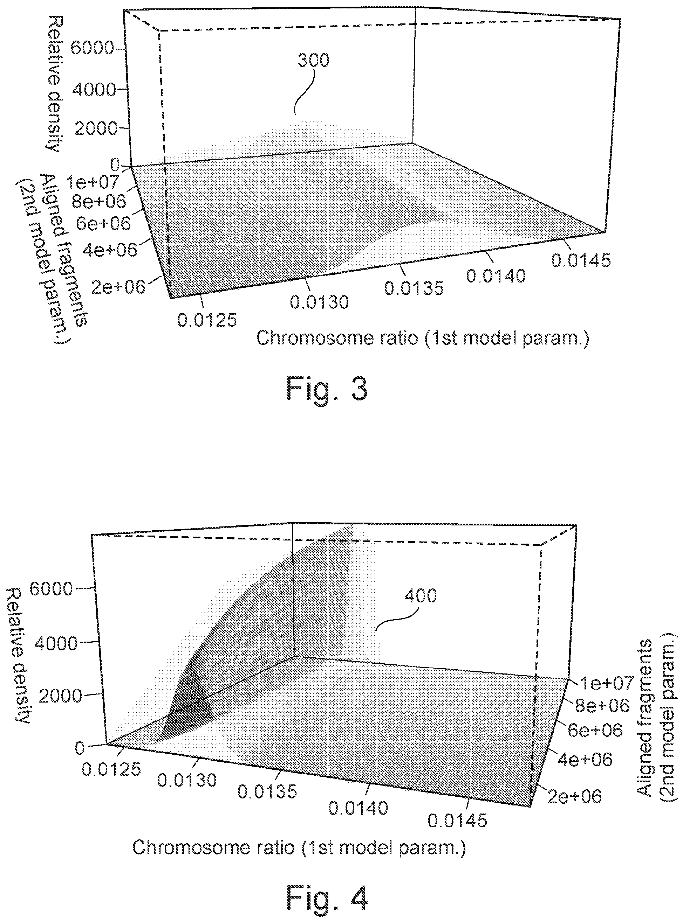

Figure 3 is an illustration 300 of the first model 121 being indicative of a probability of chromosome abnormality. Figure 4 is an illustration 400 of the second model 122 being indicative of chromosome normality. The illustrations 300, 400 are of models relating to trisomy 21, although it will be realized that this is not restrictive. Each model 121, 122 is parameterized by the chromosome count ratio, which is illustrated along the x-axis, and chromosome sequence density which may be the autosome fragment count number i.e. the total number of aligned fragments, which is illustrated along the y-axis. The first and second models 121, 122 are representative of a probability density function. The probability density functions may be determined using a combined model.

Each of the first and second models 121, 122 may determine a respective probability for parameters between minimum and maximum parameter values i.e. between minimum and maximum chromosome count ratio values and autosome fragment count numbers.

The first and second models 121, 122 may be continuous or non-continuous probability density functions i.e. the probability density may be specified by the model 121, 122 at a plurality of parameter values within the range between the minimum and maximum parameter values. For parameters values in between the specified values, the density values may be interpolated for specific parameter values. The interpolation may be a linear interpolation between adjacent specified parameter values.

Thus for the first model 121 indicative of the probability of chromosome abnormality, the density value may be determined as:

$$D_{\mathrm{Non}}(R_{in}, A)$$

where $R_{in}$ is an input chromosome count ratio and A is an input autosome fragment count number. Similarly, for the second model 122 indicative of chromosome normality the density value may be determined as:

$$D_{\mathrm{Tri}}(R_{in}, A)$$

In some embodiments, one or both of the models 121, 122 may comprise a value $R_{\mathrm{nonmean}}$ indicative of a mean unaffected chromosome count ratio for the respective chromosome or trisomy. The value of $R_{\mathrm{nonmean}}$ may be:

1. Trisomy 13: 0.037036
2. Trisomy 18: 0.030858
3. Trisomy 21: 0.012843

It will be appreciated that the above values are merely by way of example and that other values may be used.

In some embodiments, one or both of the models 121, 122 may comprise a first threshold value $R_{\mathrm{thresh1}}$. The first threshold value may be associated with the respective chromosome or trisomy. The first threshold value $R_{\mathrm{thresh1}}$ may correspond to a chromosome count ratio below which a likelihood ratio should be considered very close to zero, and set to a very small value. The value of $R_{\mathrm{thresh1}}$ may, in some embodiments, be one or more of:

1. Trisomy 13: 0.037036
2. Trisomy 18: 0.030858
3. Trisomy 21: 0.012843

It will be appreciated that the above values are merely by way of example and that other values may be used.

In some embodiments, one or both of the models 121, 122 may comprise a second threshold value $R_{\mathrm{thresh2}}$. The second threshold value may be associated with a respective chromosome or trisomy. The second threshold value $R_{\mathrm{thresh2}}$ is indicative of an affected chromosome count ratio above which a likelihood ratio should be considered effectively infinite, and set to a very large value. The value of the second threshold value $R_{\mathrm{thresh2}}$ may, in some embodiments, be one or more of:

4. Trisomy 13: 0.039153
5. Trisomy 18: 0.032622
6. Trisomy 21: 0.013577

It will be appreciated that the above values are merely by way of example and that other values may be used.

In some embodiments, one or both of the models 121, 122 may comprise an optional likelihood ratio calibration factor $k_{LR}$. The likelihood ratio calibration factor may have a default value of unity (1).

As can be appreciated particularly from Figure 4, the probability density for chromosome normality decreases noticeably with decreasing autosome count number. The decrease is particularly observable below an autosome count number of around 3 million. That is, as the number of autosome counts decreases, particularly below $3e^6$, even for the same chromosome count ratio value, the probability density value determined by the second model 122 decreases. A similar effect also occurs for the first model 121. As the probability density decreases a dispersion of each curve may increase i.e. the curve has wider tails, such that an area under each curve remains constant.

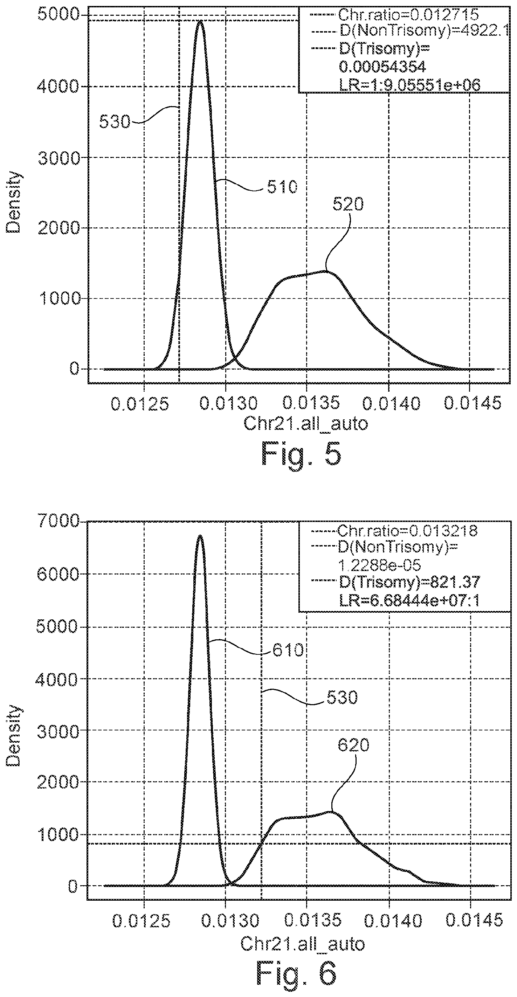

For specific input parameters, i.e. a specific count ratio for a target chromosome and a specific autosome fragment count number, a probability density value may be determined using each of the first and second models 121, 122. For example, Figures 5 and 6 illustrate probability density curves for each of the first and second models 121, 122 at first and second autosome fragment count numbers. Figure 5 is for an autosome fragment count number of 2372258 counts and Figure 6 is for an autosome fragment count number of 5588457 counts. A difference in shape between the density functions 510, 610 for the second, normality, model 122 and the density functions 520, 620 for the first, abnormality, model 121 can be observed.

As can be appreciated from Figures 5 and 6, based on the count ratio for the target chromosome at the autosome fragment count number, a probability of chromosomal normality can be determined from the normality probability distribution 510, 610 and a probability of chromosomal abnormality can be determined from the abnormality probability distribution 520, 620.

As shown in Figure 5, indicated by line 530, at a chromosome count ratio of 0.012715, i.e. approximately 1.27% of chromosome fragments being from chromosome 21, the probability density of chromosomal normality is 4922.1 whereas the probability density of chromosomal abnormality is 0.00054354.

The likelihood ratio (LR) may be determined as:

$$LR = k_{LR} \frac{D_{\mathrm{Tri}}(R_{in}, A)}{D_{\mathrm{Non}}(R_{in}, A)}$$

Thus, for Figure 5, the likelihood ratio (*LR)* is 1:9.05551e$^6$.

Referring to Figure 6, indicated by line 630, at a chromosome count ratio of 0.013218, i.e. approximately 1.32% of chromosome fragments being from chromosome 21, the probability density of chromosomal normality is 1.2288e$^{-5}$ whereas the probability density of chromosomal abnormality is 6.68444e$^7$. Therefore the *LR* is determined as 6.68444e$^7$:1.

In some embodiments, if $R_{in} > R_{\mathrm{thresh2}}$ where $R_{\mathrm{thresh2}}$ is the second threshold value, as discussed above, and $D_{\mathrm{Tri}} = 0$ then LR may be determined to have a predetermined value, such as a relatively high value. The predetermined value of *LR* may be a value representative of infinity, such as a maximum value capable of being stored by the memory unit 120, although it will be realized that other predetermined values may be used.

In some embodiments, if $R_{in} < R_{\mathrm{thresh1}}$, where $R_{\mathrm{thresh1}}$ is the first threshold value, as discussed above, and $D_{\mathrm{Non}} = 0$ then LR may be determined to have a predetermined value, such as a relatively low value. The predetermined value of *LR* may be a value of zero. The predetermined value of *LR* may be a minimum value capable of being stored by the memory unit 120, although it will be realized that other predetermined values may be used. The value may be a minimum non-zero value.

If neither of the above first and/or second threshold situations occur, and *LR* is undefined due to a zero-valued $D_{\mathrm{Non}}$ then, in some embodiments, LR may be determined to be a maximum machine representable value which may be stored in the memory unit 120.

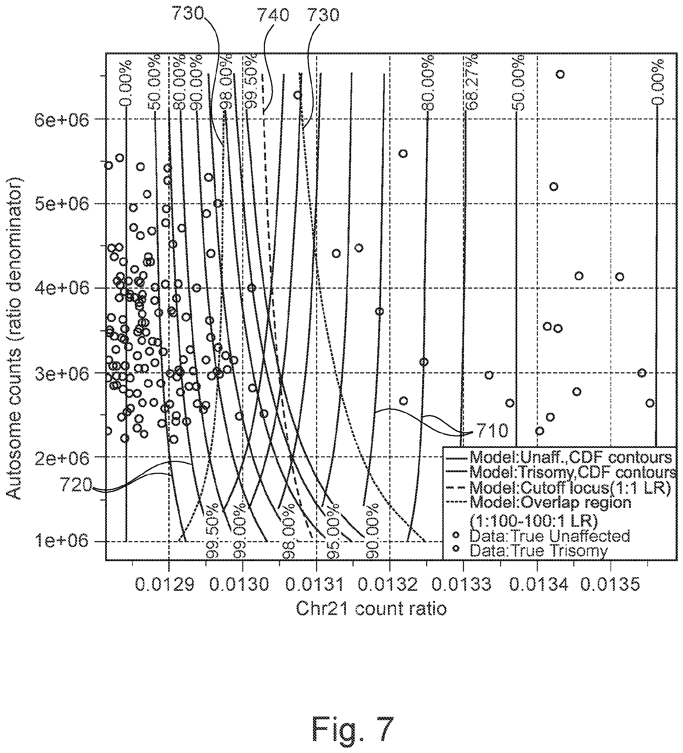

Figure 7 illustrates density contours based on the first and second models 121, 122. For each model 121, 122 density contours are illustrated representative of a plurality of specific densities. The specific density contours illustrated in Figure 7 enclose 50, 80, 90 and 99.5% of the total density for each model 121, 122. Density contours 710 for the first model 121 indicative of a probability of chromosome abnormality and the second model 122 indicative of a probability of chromosome normality are illustrated, although not all are labeled in Figure 7 for clarity. An overlap region is indicated in Figure 7 with dotted border lines 730. The overlap region is indicative of a region for which the first and second models 121, 122 overlap. The overlap is between first and second predetermined likelihood ratios which, in Figure 7, are 1:100 and 100:1 although the overlap region may be determined for other predetermined likelihood ratios. Similarly, a line of unity likelihood ratio (1:1) is indicated with reference 740. As can be appreciated, the overlap region has curved boundary lines and the line of unity LR 740 is also curved. In particular it can be appreciated that the overlap region widens with decreasing autosome count numbers. Thus Figure 7 illustrates the influence of changing autosome count number and chromosome count ratio on determined density and LR. Although Figure 7 relates to chromosome 21, it will be realised that this is not limiting.

Returning to Figure 2, in step 230 a performance parameter threshold is determined for reducing a number of false results. In one embodiment the determined performance parameter is a fetal fraction threshold (FFT). In some embodiments the FFT is referred to as $F_{\mathrm{FN\_min}}$. The FFT is used to reduce numbers of false negative results i.e. where a test is incorrectly determined to be negative for chromosomal abnormality. The FFT is determined dynamically in embodiments of the invention. The dynamic determination is to avoid use of a predetermined i.e. fixed fetal fraction threshold which may cause excessive numbers of tests to be declared as unreliable. The dynamic determination of FFT is based upon the second parameter representative of chromosome sequence density such as the autosome fragment count or other parameter indicative of sequencing coverage.

An estimate of the performance parameter is provided by the DNA processing system. In particular, in some embodiments, an estimate of fetal fraction $\hat{F}$ is determined by the DNA processing system 150. The fetal fraction estimate is an estimate of a fraction of autosome fragments originating from the foetus. Since embodiments of the invention aim to determine chromosomal abnormality of the foetus it is necessary that sufficient autosome fragments originate from the foetus rather than the mother. The estimate of fetal fraction may be accompanied by an indication of an uncertainty value associated with the estimate.

A minimum count ratio $R_{\mathrm{FN\_min}}$ needed to support dynamic fetal fraction estimation is determined as the value of count ratio below which the proportional area under normality probability density function $D_{\mathrm{Non}}(R_{in},A)$ from the first model 121 is a predetermined minimum sensitivity, *MinSensitivity* and, as above, A is the total autosome fragment count. The predetermined minimum sensitivity, M*inSensitivity,* may be determined for each respective chromosome or trisomy. M*inSensitivity* may be:

- Trisomy 13: 0.80
- Trisomy 18: 0.98
- Trisomy 21: 0.99

although it will be realised that other values may be selected. The minimum count ratio $R_{\mathrm{FN\_min}}$ may be converted to a minimum fetal fraction value, as follows:

$$F_{\mathrm{FN\_min}} = 2\left(\frac{R_{\mathrm{FN\_min}}}{R_{\mathrm{non_{mean}}}} - 1\right) + F_{\mathrm{offs}}.$$

Where, as above, $R_{\mathrm{non_{mean}}}$ is indicative of a mean unaffected chromosome count ratio for the respective chro-

mosome or trisomy.

$F_{\text{offs}}$ is a fetal fraction validity test threshold offset and may be defined as:

$$F_{\text{offs}} = a_{\text{FT}} + b_{\text{FT}} \sigma_F$$

where $\sigma_F$ is a standard deviation of the fetal fraction estimation distribution produced by the fetal fraction estimation stage, and $a_{\text{FT}}$ and $b_{\text{FT}}$ are validity test threshold determination parameters. The standard deviation of the fetal fraction estimation distribution may have been determined beforehand, such as to fit an empirical error distribution. These determination parameters may have initial values of one or both of $a_{\text{FT}} = 0$ and

$$b_{\text{FT}} = 1.96\sqrt{2}$$ although it will be realised that other initial values may be used.

In step 230 it is determined whether the estimate of fetal fraction F̂ for the sample is less than the dynamic FFT $F_{\text{FN\_min}}$. In some embodiments, the fetal fraction estimate is scaled according to a number of foetus. Step 230 may comprise determining whether:

$$\frac{\widehat{F}}{\tau} < F_{\text{FN\_min}}$$

where $\tau$ is a multiple pregnancy correction. If the biological sample is a sample from a multiple pregnancy, $\tau$ should be equal to the number of fetuses carried in the pregnancy, otherwise it should be unity.

If it is determined in step 240 that F̂ or $\frac{\widehat{F}}{\tau}$ is less than the threshold, then it is determined that the test may be unreliable and the method 200 moves to step 250. In step 250 a suitable warning may be output indicative of the low fetal fraction in the biological sample. Otherwise, in step 260 an output of the test may be output. The output may be the *LR* determined for the sample.

Advantageously, embodiments of the invention afford a more efficient use of data than, for example, pre-filtering approaches, by allowing accurate test results to be generated where a fragment count or fetal fraction test would have rejected the test results. For example, embodiments of the invention produce a result where a fragment count proportion represents a sufficiently definitive 'positive' result despite a low fragment count or low fetal fraction.

It will be appreciated that embodiments of the present invention can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement embodiments of the present invention. Accordingly, embodiments provide a program comprising code for implementing a system or method as claimed in any preceding claim and a machine readable storage storing such a program. Still further, embodiments of the present invention may be conveyed electronically via any medium such as a communication signal carried over a wired or wireless connection and embodiments suitably encompass the same.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed. The claims should not be construed to cover merely the foregoing embodiments, but also any embodiments which fall within the scope of the claims.

**Claims**

1. A computer-implemented method of determining a probability of a fetal chromosomal abnormality, the method comprising:

> determining a first parameter corresponding to a chromosome count ratio for a target chromosome, wherein the chromosome count ratio is a ratio of a total number of matched nucleic acids for the target chromosome relative to a total number of matched nucleic acids assigned to each of one or more reference chromosomes, and a second parameter corresponding to a measure of sequencing coverage of the one or more reference chromosomes from a biological sample obtained from a female subject;
> determining a likelihood ratio indicative of fetal chromosomal abnormality, wherein the likelihood ratio is determined as a ratio between a probability of chromosomal abnormality and a probability of chromosomal normality according to respective abnormality and normality models based on the first and second parameters, wherein the abnormality model is representative of a probability density function indicative of chromosome abnormality and the normality model is representative of a probability density function indicative of chromosome normality; determining one or more performance parameter thresholds wherein the performance parameter is indicative of a fraction of autosome fragments originating from a foetus and the performance parameter threshold is determined based on the second parameter representative of chromosome sequence density; and comparing an estimate of one or more performance parameters associated with the sample against the one or more performance parameter thresholds to determine a reliability of the likelihood ratio and outputting the likelihood ratio in dependence thereon.

2. The method of claim 1, wherein at least one of the performance parameter thresholds is determined from the normality model.

3. The method of claim 1, wherein the one or more performance parameter thresholds comprise a fetal fraction threshold optionally the fetal fraction threshold is determined based upon the normality model.

4. The method of claim 3, wherein said comparing comprises comparing the estimate of fetal fraction for the sample against said the fetal fraction threshold.

5. The method of any preceding claim comprising determining said biological sample as having a low performance parameter when the estimate of the performance parameter is less than the performance parameter threshold; optionally the method comprises determining said biological sample as having a low fetal fraction when the estimate of fetal fraction is less than the fetal fraction threshold.

6. The method of any preceding claim, wherein the performance parameter threshold is dynamically determined based upon the first and second parameters

7. The method of claim 6 when dependent on claim 3 or any claim dependent thereon, wherein: the fetal fraction threshold is dynamically determined based upon the first and second parameters; and/or the fetal fraction threshold is determined based a minimum count ratio.

8. The method of claim 7, wherein the minimum count ratio is based on an area under a probability density function from the normality model being a predetermined minimum sensitivity.

9. The method of claim 3 or any claim dependent thereon, wherein the fetal fraction threshold $F_{FN\_min}$ is determined as:

$$F_{FN\_min} = 2\left(\frac{R_{FN\_min}}{R_{non_{mean}}} - 1\right)$$

where $R_{FN\_min}$ is a minimum count ratio and $R_{non_{mean}}$ is a mean unaffected chromosome count ratio for the target chromosome.

10. The method of any preceding claim, wherein the likelihood ratio ($LR$) is determined as:

$$LR = \frac{D_{Tri}(R_{in}, A)}{D_{Non}(R_{in}, A)}$$

where $D_{Tri}(R_{in}, A)$ is a probability of chromosomal abnormality determined according to the abnormality model and $D_{Non}(R_{in}, A)$ is a probability of chromosomal normality determined according to the normality model, $R_{in}$ is the first parameter for the target chromosome and A is the second parameter representative of chromosome sequence density.

11. The method of any preceding claim, wherein the chromosome count ratio is a ratio of the total number of matched nucleic acids assigned to a target chromosome relative to the total number of matched nucleic acids assigned to each of one or more reference chromosomes.

12. The method of any preceding claim, wherein the second parameter is indicative of an autosome count number.

13. An apparatus, comprising:

a processing unit; and
a memory unit storing data representing abnormality and normality models and computer executable instructions which, when executed by the processor perform the steps of:

determining a first parameter corresponding to a chromosome count ratio for a target chromosome, wherein the chromosome count ratio is a ratio of a total number of matched nucleic acids for the target chromosome relative to a total number of matched nucleic acids assigned to each of one or more reference chromosomes, and a second parameter corresponding to a measure of sequencing coverage of the one or more reference chromosomes from a biological sample obtained from a female subject;

determining a likelihood ratio indicative of fetal chromosomal abnormality, wherein the likelihood ratio is determined as a ratio between a probability of chromosomal abnormality and a probability of chromosomal normality according to the respective abnormality and normality models based on the first and second parameters, wherein the abnormality model is representative of a probability density function indicative of chromosome abnormality and the normality model is representative of a probability density function indicative of chromosome normality; and

determining one or more performance parameter thresholds wherein the performance parameter is indicative of a fraction of autosome fragments originating from a foetus and the performance parameter threshold is determined based on the second parameter representative of chromosome sequence density;

comparing an estimate of one or more performance parameters associated with the sample against the one or more performance parameter thresholds to determine a reliability of the likelihood ratio and outputting the likelihood ratio in dependence thereon.

14. The apparatus of claim 13 comprising an interface unit for receiving data from a DNA processing system.

15. Computer software which, when executed by a computer, is arranged to perform a method as claimed in any of claims 1 to 12; optionally the computer software is stored on a computer-readable medium.

**Patentansprüche**

1. Computerimplementiertes Verfahren zum Bestimmen einer Wahrscheinlichkeit einer fetalen Chromosomenanomalie, wobei das Verfahren Folgendes umfasst:

Bestimmen eines ersten Parameters, der einem Chromosomenzahlverhältnis für ein Zielchromosom entspricht, wobei das Chromosomenzahlverhältnis ein Verhältnis einer Gesamtanzahl von übereinstimmenden Nukleinsäuren für das Zielchromosom relativ zu einer Gesamtanzahl von übereinstimmenden Nukleinsäuren ist, die jedem von einem oder mehreren Referenzchromosomen zugeordnet sind, und eines zweiten Parameters, der einem Maß der Sequenzabdeckung des einen oder der mehreren Referenzchromosomen aus einer biologischen Probe entspricht, die von einem weiblichen Subjekt erhalten wurde;

Bestimmen eines Wahrscheinlichkeitsverhältnisses, das eine fetale Chromosomenanomalie angibt, wobei das Wahrscheinlichkeitsverhältnis als ein Verhältnis zwischen einer Wahrscheinlichkeit einer Chromosomenanomalie und einer Wahrscheinlichkeit einer Chromosomennormalität gemäß jeweiligen Anomalie- und Normalitätsmodellen auf Grundlage des ersten und des zweiten Parameters bestimmt wird, wobei das Anomaliemodell eine Wahrscheinlichkeitsdichtefunktion darstellt, die eine Chromosomenanomalie angibt, und das Normalitätsmodell eine Wahrscheinlichkeitsdichtefunktion darstellt, die eine Chromosomennormalität angibt;

Bestimmen eines oder mehrerer Leistungsparameterschwellenwerte, wobei der Leistungsparameter eine Fraktion von Autosomenfragmenten angibt, die von einem Fetus stammt, und der Leistungsparameterschwellenwert basierend auf dem zweiten Parameter, der die Chromosomsequenzdichte darstellt, bestimmt wird; und

Vergleichen einer Schätzung eines oder mehrerer Leistungsparameter, die der Probe zugeordnet sind, mit dem einen oder den mehreren Leistungsparameterschwellenwerten, um eine Zuverlässigkeit des Wahrscheinlichkeitsverhältnisses zu bestimmen, und Ausgeben des Wahrscheinlichkeitsverhältnisses in Abhängigkeit davon.

2. Verfahren nach Anspruch 1, wobei mindestens einer der Leistungsparameterschwellenwerte aus dem Normalitätsmodell bestimmt wird.

3. Verfahren nach Anspruch 1, wobei der eine oder die mehreren Leistungsparameterschwellenwerte einen Schwellenwert für die fetale Fraktion umfassen, optional wobei der Schwellenwert für die fetale Fraktion basierend auf dem Normalitätsmodell bestimmt wird.

4. Verfahren nach Anspruch 3, wobei das Vergleichen Vergleichen der Schätzung der fetalen Fraktion für die Probe mit dem Schwellenwert für die fetale Fraktion umfasst.

5. Verfahren nach einem vorhergehenden Anspruch, umfassend Bestimmen, dass die biologische Probe einen niedrigen Leistungsparameter aufweist, wenn die Schätzung des Leistungsparameters kleiner als der Leistungsparameterschwellenwert ist; wobei das Verfahren optional Bestimmen, dass die biologische Probe eine niedrige fetale Fraktion aufweist, wenn die Schätzung der fetalen Fraktion kleiner als der Schwellenwert für die fetale Fraktion ist, umfasst.

6. Verfahren nach einem vorhergehenden Anspruch, wobei der Leistungsparameterschwellenwert basierend auf dem ersten und dem zweiten Parameter dynamisch bestimmt wird.

7. Verfahren nach Anspruch 6, wenn abhängig von Anspruch 3 oder einem davon abhängigen Anspruch, wobei:
der Schwellenwert für die fetale Fraktion basierend auf dem ersten und dem zweiten Parameter dynamisch bestimmt wird; und/oder der Schwellenwert für die fetale Fraktion basierend auf einem Mindestzahlverhältnis bestimmt wird.

8. Verfahren nach Anspruch 7, wobei das Mindestzahlverhältnis darauf basiert, dass eine Fläche unter einer Wahrscheinlichkeitsdichtefunktion aus dem Normalitätsmodell eine vorbestimmte Mindestempfindlichkeit ist.

9. Verfahren nach Anspruch 3 oder einem davon abhängigen Anspruch, wobei der Schwellenwert für die fetale Fraktion $F_{\text{FN\_min}}$ bestimmt wird als:

$$F_{\text{FN\_min}} = 2\left(\frac{R_{\text{FN min}}}{R_{\text{non}_{\text{mean}}}} - 1\right)$$

wobei $R_{\text{FN\_min}}$ ein Mindestzahlverhältnis ist und $R_{\text{non}_{\text{mean}}}$ ein mittleres Zahlverhältnis nicht betroffener Chromosomen für das Zielchromosom ist.

10. Verfahren nach einem vorhergehenden Anspruch, wobei das Wahrscheinlichkeitsverhältnis (LR) bestimmt wird als:

$$LR = \frac{D_{\text{Tri}}(R_{in}, A)}{D_{\text{Non}}(R_{in}, A)}$$

wobei $D_{\text{Tri}}(R_{in}, A)$ eine Wahrscheinlichkeit einer Chromosomenanomalie ist, die gemäß dem Anomaliemodell bestimmt wurde, und $D_{\text{Non}}(R_{in}, A)$ eine Wahrscheinlichkeit einer Chromosomennormalität ist, die gemäß dem Normalitätsmodell bestimmt wurde, $R_{in}$ der erste Parameter für das Zielchromosom ist und A der zweite Parameter ist, der die Chromosomensequenzdichte darstellt.

11. Verfahren nach einem vorhergehenden Anspruch, wobei das Chromosomenzahlverhältnis ein Verhältnis der Gesamtanzahl von übereinstimmenden Nukleinsäuren ist, die einem Zielchromosom zugeordnet sind, relativ zu der Gesamtanzahl von übereinstimmenden Nukleinsäuren, die jedem von einem oder mehreren Referenzchromosomen zugeordnet sind.

12. Verfahren nach einem vorhergehenden Anspruch, wobei der zweite Parameter eine Autosomenzahlzahl angibt.

13. Vorrichtung, umfassend:

eine Verarbeitungseinheit; und
eine Speichereinheit, die Daten, die Anomalie- und Normalitätsmodelle darstellen, und computerausführbare Anweisungen speichert, die, wenn sie durch den Prozessor ausgeführt werden, die folgenden Schritte durchführen:

Bestimmen eines ersten Parameters, der einem Chromosomenzahlverhältnis für ein Zielchromosom entspricht, wobei das Chromosomenzahlverhältnis ein Verhältnis einer Gesamtanzahl von übereinstimmenden Nukleinsäuren für das Zielchromosom relativ zu einer Gesamtanzahl von übereinstimmenden Nukleinsäuren ist, die jedem von einem oder mehreren Referenzchromosomen zugeordnet sind, und eines zweiten Parameters, der einem Maß der Sequenzabdeckung des einen oder der mehreren Referenzchromosomen aus einer biologischen Probe entspricht, die von einem weiblichen Subjekt erhalten wurde;
Bestimmen eines Wahrscheinlichkeitsverhältnisses, das eine fetale Chromosomenanomalie angibt, wobei das Wahrscheinlichkeitsverhältnis als ein Verhältnis zwischen einer Wahrscheinlichkeit einer Chromosomenanomalie und einer Wahrscheinlichkeit einer Chromosomennormalität gemäß den jeweiligen Anomalie- und Normalitätsmodellen auf Grundlage des ersten und des zweiten Parameters bestimmt wird, wobei das Anomaliemodell eine Wahrscheinlichkeitsdichtefunktion darstellt, die eine Chromosomenanomalie angibt, und das Normalitätsmodell eine Wahrscheinlichkeitsdichtefunktion darstellt, die eine Chromosomennormalität angibt; und
Bestimmen eines oder mehrerer Leistungsparameterschwellenwerte, wobei der Leistungsparameter eine Fraktion von Autosomenfragmenten angibt, die von einem Fetus stammt, und der Leistungsparameterschwellenwert basierend auf dem zweiten Parameter, der die Chromosomsequenzdichte darstellt, bestimmt wird;
Vergleichen einer Schätzung eines oder mehrerer Leistungsparameter, die der Probe zugeordnet sind, mit dem einen oder den mehreren Leistungsparameterschwellenwerten, um eine Zuverlässigkeit des Wahrscheinlichkeitsverhältnisses zu bestimmen, und Ausgeben des Wahrscheinlichkeitsverhältnisses in Abhängigkeit davon.

14. Vorrichtung nach Anspruch 13, umfassend eine Schnittstelleneinheit zum Empfangen von Daten von einem DNA-Verarbeitungssystem.

15. Computersoftware, die bei Ausführung durch einen Computer dazu ausgelegt ist, ein Verfahren wie nach einem der Ansprüche 1 bis 12 beansprucht durchzuführen; wobei die Computersoftware optional auf einem computerlesbaren Medium gespeichert ist.

**Revendications**

1. Procédé mise en œuvre par ordinateur permettant la détermination d'une probabilité d'anomalie chromosomique fœtale, le procédé comprenant :

la détermination d'un premier paramètre correspondant au rapport de numération des chromosomes pour un chromosome cible, ledit rapport de numération des chromosomes étant un rapport d'un nombre total d'acides nucléiques appariés pour le chromosome cible par rapport à un nombre total d'acides nucléiques appariés attribués à chacun d'un ou plusieurs chromosomes de référence, et un second paramètre correspondant à une mesure de couverture de séquençage desdits un ou plusieurs chromosomes de référence à partir d'un échantillon biologique obtenu auprès d'un sujet femelle ;
la détermination d'un rapport de probabilité indiquant une anomalie chromosomique fœtale, ledit rapport de probabilité étant déterminé sous la forme d'un rapport entre une probabilité d'anomalie chromosomique et une probabilité de normalité chromosomique conformément à des modèles d'anomalie et de normalité respectifs basés sur les premier et second paramètres, ledit modèle d'anomalie représentant une fonction de densité de probabilité indiquant une anomalie chromosomique et ledit modèle de normalité représentant une fonction de densité de probabilité indiquant une normalité chromosomique ;
la détermination d'un ou plusieurs seuils de paramètre de performance, ledit paramètre de performance indiquant une fraction de fragments d'autosome provenant d'un fœtus et ledit seuil de paramètre de performance étant déterminé sur la base du second paramètre représentant la densité de séquence chromosomique ; et
la comparaison d'une estimation d'un ou plusieurs paramètres de performance associés à l'échantillon avec lesdits un ou plusieurs seuils de paramètre de performance pour déterminer une fiabilité du rapport de probabilité et émettre en sortie le rapport de probabilité en fonction de celle-ci.

2. Procédé selon la revendication 1, au moins l'un des seuils de paramètre de performance étant déterminé à partir du modèle de normalité.

3. Procédé selon la revendication 1, lesdits un ou plusieurs seuils de paramètre de performance comprenant un seuil de fraction fœtale, éventuellement ledit seuil de fraction fœtale étant déterminé sur la base du modèle de normalité.

4. Procédé selon la revendication 3, ladite comparaison comprenant la comparaison de l'estimation de la fraction fœtale pour l'échantillon avec ledit seuil de fraction fœtale.

5. Procédé selon l'une quelconque des revendications précédentes, comprenant la détermination dudit échantillon biologique comme présentant un paramètre de performance faible lorsque l'estimation du paramètre de performance est inférieure au seuil de paramètre de performance ; éventuellement, le procédé comprenant la détermination dudit échantillon biologique comme présentant une fraction fœtale faible lorsque l'estimation de la fraction fœtale est inférieure au seuil de fraction fœtale.

6. Procédé selon l'une quelconque des revendications précédentes, ledit seuil de paramètre de performance étant déterminé de manière dynamique sur la base des premier et second paramètres.

7. Procédé selon la revendication 6 lorsqu'elle dépend de la revendication 3 ou toute revendication qui en dépend :
ledit seuil de fraction fœtale étant déterminé de manière dynamique sur la base des premier et second paramètres ; et/ou ledit seuil de fraction fœtale étant déterminé sur la base d'un rapport de numération minimal.

8. Procédé selon la revendication 7, ledit rapport de numération minimal étant basé sur une zone sous une fonction de densité de probabilité à partir du fait que le modèle de normalité est une sensibilité minimale prédéfinie.

**9.** Procédé selon la revendication 3 ou toute revendication qui en dépend, ledit seuil de fraction fœtale $F_{FN\_min}$ étant déterminé comme étant :

$$F_{FN\_min} = 2\left(\frac{R_{FN\ min}}{R_{non_{mean}}} - 1\right)$$

où $R_{FN\_min}$ représente un rapport de numération minimal et $R_{non_{mean}}$ représente un rapport de numération des chromosomes moyen non affecté pour le chromosome cible.

**10.** Procédé selon l'une quelconque des revendications précédentes, ledit rapport de probabilité (LR) étant déterminé comme étant :

$$LR = \frac{D_{Tri}(R_{in}, A)}{D_{Non}(R_{in}, A)}$$

où $D_{Tri}(R_{in}, A)$ représente une probabilité d'anomalie chromosomique déterminée selon le modèle d'anomalie et $D_{Non}(R_{in}, A)$ représente une probabilité de normalité chromosomique déterminée selon le modèle de normalité, $R_{in}$ représente le premier paramètre pour le chromosome cible et A représente le second paramètre représentant la densité de séquence chromosomique.

**11.** Procédé selon l'une quelconque des revendications précédentes, ledit rapport de numération des chromosomes étant un rapport du nombre total d'acides nucléiques appariés attribués à un chromosome cible par rapport au nombre total d'acides nucléiques appariés attribués à chacun d'un ou plusieurs chromosomes de référence.

**12.** Procédé selon l'une quelconque des revendications précédentes, ledit second paramètre indiquant un nombre de numération des autosomes.

**13.** Appareil, comprenant :

une unité de traitement ; et
une unité de mémoire stockant des données représentant des modèles d'anomalie et de normalité et des instructions exécutables par ordinateur qui, lorsqu'elles sont exécutées par le processeur, effectuent les étapes de :

détermination d'un premier paramètre correspondant au rapport de numération des chromosomes pour un chromosome cible, ledit rapport de numération des chromosomes étant un rapport d'un nombre total d'acides nucléiques appariés pour le chromosome cible par rapport à un nombre total d'acides nucléiques appariés attribués à chacun d'un ou plusieurs chromosomes de référence, et un second paramètre correspondant à une mesure de couverture de séquençage desdits un ou plusieurs chromosomes de référence à partir d'un échantillon biologique obtenu auprès d'un sujet femelle ;

détermination d'un rapport de probabilité indiquant une anomalie chromosomique fœtale, ledit rapport de probabilité étant déterminé sous la forme d'un rapport entre une probabilité d'anomalie chromosomique et une probabilité de normalité chromosomique conformément aux modèles d'anomalie et de normalité respectifs basés sur les premier et second paramètres, ledit modèle d'anomalie étant représentatif d'une fonction de densité de probabilité indiquant une anomalie chromosomique et ledit modèle de normalité étant représentatif d'une fonction de densité de probabilité indiquant une normalité chromosomique ; et

détermination d'un ou plusieurs seuils de paramètre de performance, ledit paramètre de performance indiquant une fraction de fragments d'autosome provenant d'un fœtus et ledit seuil de paramètre de performance étant déterminé sur la base du second paramètre représentant la densité de séquence chromosomique ;

comparaison d'une estimation d'un ou plusieurs paramètres de performance associés à l'échantillon avec lesdits un ou plusieurs seuils de paramètre de performance pour déterminer une fiabilité du rapport de probabilité et émettre en sortie le rapport de probabilité en fonction de celle-ci.

**14.** Appareil selon la revendication 13, comprenant une unité d'interface destinée à recevoir des données d'un système de traitement d'ADN.

**15.** Logiciel informatique qui, lorsqu'il est exécuté par un ordinateur, est agencé pour réaliser un procédé selon l'une quelconque des revendications 1 à 12 ; éventuellement, le logiciel informatique est stocké sur un support lisible par ordinateur.

Fig. 1

Fig. 2

Fig. 3

Fig. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010112590 A1
- US 2015267255 A1

- WO 2014033455 A

**Non-patent literature cited in the description**

- **A. J. SEHNERT**. Optimal Detection of Fetal Chromosomal Abnormalities. *Massively Parallel DNA Sequencing of Cell-Free Fetal DNA from Maternal Blood*, 01 July 2011

- **H. CHRISTINA FAN**. *Sensitivity of Noninvasive Prenatal Detection of Fetal Aneuploidy from Maternal Plasma Using Shotgun Sequencing Is Limited Only by Counting Statistics*, 01 January 2010